# EUROPEAN PATENT SPECIFICATION

(11) **EP 0 648 123 B1**
(45) Date of publication and mention of the grant of the patent: **09.10.1996**
(21) Application number: 93915598.2
(22) Date of filing: 09.07.1993
(51) Int. Cl.: A61K 33/24, A61K 39/395, A61K 31/70, A61K 48/00

(54) **INHIBITORS OF PROTEIN TYROSINE-PHOSPHATASE $g(a) FOR TREATMENT OF TUMORS**
Proteintyrosinphosphatase alpha - Inhibitoren zur Tumorbehandlung
INHIBITEURS DE TYROSINE PHOSPHATASE $g(a) PROTEIQUE POUR LE TRAITEMENT DES TUMEURS

(30) Priority: 10.07.1992 GB 9214754
(43) Date of publication of application: 19.04.1995
(73) Proprietor: NATIONAL UNIVERSITY OF SINGAPORE, Singapore 0511 (SG)
(72) Inventor: PALLEN, Catherine Jane, Institute of Molecular and, 10 Kent Ridge Crescent, Singapore 119260 (SG); ZHENG, Xin Min, Institute of Molecular and, 10 Kent Ridge Crescent, Singapore 119260 (SG); WANG, Yue, Institute of Molecular and Cell Biology, 10 Kent Ridge Crescent, Singapore 119260 (SG)
(74) Representative: Woods, Geoffrey Corlett
(86) International application number: CA9300285
(87) International publication number: WO9401119

(56) References cited:
- EP-A- 0 245 979
- WO-A-91/13989
- WO-A-92/01050
- EMBO JOURNAL vol. 10, no. 11, November 1991, OXFORD, GB pages 3231 - 3237 Y. WANG ET AL. 'The receptor-like protein tyrosine phosphatase HPTPalpha has two active catalytic domains with distinct substrate specificities.'
- NATURE vol. 359, no. 6393, 24 September 1992, LONDON, GB pages 336 - 339 X. ZHENG ET AL. 'Cell transformation and activation of pp60c-src by overexpression of a protein tyrosine phosphatase.'

## Description

This invention relates to the treatment of tumours, in particular tumours exhibiting an elevated level of pp60^{c-src} kinase activity such as human colon carcinoma.

The kinase activity of pp60^{c-src} is specifically and transiently increased during mitosis and repressed during interphase¹. Loss of cell cycle control of pp60^{c-src} occurs upon mutation of Tyr 527 to Phe or when pp60^{c-src} is associated with polyoma middle-T-antigen, and these conditions result in cell transformation or tumourigenesis ^{2,3}. In both cases pp60^{c-src} has elevated kinase activity which is maintained throughout the cell cycle and accompanied by dephosphorylation of the carboxy-terminal negative regulatory ⁴⁻⁷ Tyr 527 site, or mimicry of Tyr 527 dephosphorylation in the case of the mutant.

Protein tyrosine phosphatases (PTPases) have been considered as potential anti-oncogenes ^{8,9}, where mutations giving rise to a non-functional gene product or gene deletion could result in tumourigenesis. We have now found that over-expression of the receptor-like human PTPα (HPTPα) results in persistent activation of pp60^{c-src} with concomitant cell transformation and tumourigenesis. This indicates instead that PTPα may function as an oncogene and not an anti-oncogene.

According to the present invention, there is provided an inhibitor of PTPα for use in the treatment of a tumour exhibiting an elevated level of pp60^{c-src} kinase activity.

PTPases may be more fully termed as protein tyrosine phosphate phosphohydrolases (EC 3.1.3.48). They may be divided into three classes based on their structural organisation. Class I contains the low molecular mass non-receptor molecules possessing a single catalytic domain (PTP IB, TCPTP, rat brain PTP). Class II and II PTPases are receptor-like transmembrane proteins. The sole member of Class II is human PTPβ (HPTPβ) which has a single cytoplasmic catalytic domain. Class III members are LCA, LAR, HPTPα, HPTPγ, HPTPδ, HPTPε, DPTP, DLAR and possess two repeated putative catalytic domains in the cytoplasmic region of the molecule.

Inhibitors of PTPα include chemical compounds, for example zinc ions, vanadates such as sodium orthovanadate and arsenites such as phenylarsine oxide. These compounds are, however, fairly toxic. Non-toxic inhibitors are preferred. The inhibitor may therefore be an antibody, polyclonal or monoclonal. The antibody is typically an inhibitory antibody to the extracellular domain of PTPα or to the active site in the catalytic domain of PTPα.

Inhibitors such as zinc, vanadate, arsenite and antibody act directly on PTPα. However, inhibition may be achieved in other ways. Inhibition of endogenous PTPα may be effected by gene transfer of a mutant PTPα cDNA where the mutant protein product interacts with and blocks endogenous PTPα activity or competes with endogenous PTPα for activating proteins, ligands, etc. A suitable cDNA encodes a mutant PTPα in which the Cys residues of the catalytic domain(s) have been mutated to Ser.

The inhibitors are used to treat a tumour exhibiting an elevated level of pp60^{c-src} kinase activity. Any tumour which has abnormally active or overactive pp60^{c-src}, which may be a result of PTPα overexpression or overactivation in the tumour, may be treated. The tumour may be a tumour with increased pp60^{c-src} activity which cannot be accounted for by a proportional increase in pp60^{c-src} amount. Such tumours include human colon carcinoma, rhabdomyosarcoma, osteogenic sarcoma and Ewing's sarcoma ¹⁰⁻¹⁴. The inhibitors may be particularly used in the treatment of human colon carcinoma which is the third most common human malignancy. The inhibitors may therefore be used in the treatment of colorectal cancer.

An agent for use in the treatment of a tumour exhibiting an elevated level of pp60^{c-src} kinase activity can therefore be provided. The agent comprises a HPTPα inhibitor. A pharmaceutical composition may be formulated, comprising the inhibitor and a pharmaceutically acceptable carrier or diluent.

A chemical or antibody inhibitor may be given by injection. When an injectable preparation is produced, a pH controlling agent, a buffer, a stabilizing agent and/or an excipient may be added. Further, according to conventional techniques, lyophilized injectable preparations may be produced. There can be produced preparations for subcutaneous, intramuscular or intravenous injection.

A chemical inhibitor may alternatively be administered by the oral or rectal route. Orally administrable solid preparations may be produced by adding an excipient and, if desired, a binding agent, disintegrator, lubricating agent, coloring agent, and/or flavouring agent, and the like to the inhibitor, and forming tablets, coated tablets, granules, powders or capsules according to conventional techniques. Oral liquid compositions may be prepared by adding a flavouring agent, buffer, and/or stabilizer to the main drug and forming a syrup or dry syrup. A rectal suppository may be prepared by adding an excipient and, if desired, a surfactant to the inhibitor to form the rectal suppository according to conventional techniques.

A DNA sequence encoding a mutant PTPα may be incorporated in a retroviral or adenoviral vector or in liposomes for the purpose of gene transfer¹⁵. The vectors may transfer the DNA into cultured human tumour cells which are administered to a patient by injection, for example intratumoural injection. Cells from the tumour it is wished to treat may therefore be removed from a patient, grown in culture, infected with the vector, selected for positive infection and then reintroduced into the patient. Liposomes containing DNA, for example plasmid DNA, are injected directly into tumour cells to accomplish gene transfer. In an alternative embodiment, physical DNA transfer may be achieved using biolistic technology i.e. a gene gun.

In use, a therapeutically effective amount of the inhibitor is administered to a host having a tumour exhibiting an elevated level of pp60^{c-src} kinase activity. The host is typically a human but may be another mammal. The condition of a patient may thus be improved. The tumour from which a patient suffers may be ameliorated. This treatment may be given in conjunction with another method of treatment, for example surgery, to eradicate the tumour.

The dosage levels for the inhibitor differ depending on a variety of factors including condition of the patient, the age and body weight of the patient, the disease state being treated and the administration form. Dosage levels of the order of about 0.05-500 mg per day are useful for injection of a chemical or antibody inhibitor though the dosage levels may differ from portion to portion. When a chemical inhibitor is administered orally or rectally, the dosage levels are about 0.05-1000 mg per day. The dosage levels may be changed according to the requirements of a doctor.

For the purpose of gene transfer, enough DNA is used to introduce at least one gene per target cell such as 1 to 10000 DNA molecules per cell. The amount of DNA used depends on the tumour mass and estimated number of cells per tumour. It also depends on the amount of endogenous PTPα per tumour cell. Generally an amount of inhibitory mutant PTPα DNA is introduced that gives expression of an excess amount of mutant PTPα protein.

The following Examples illustrate the invention. In the accompanying drawings:

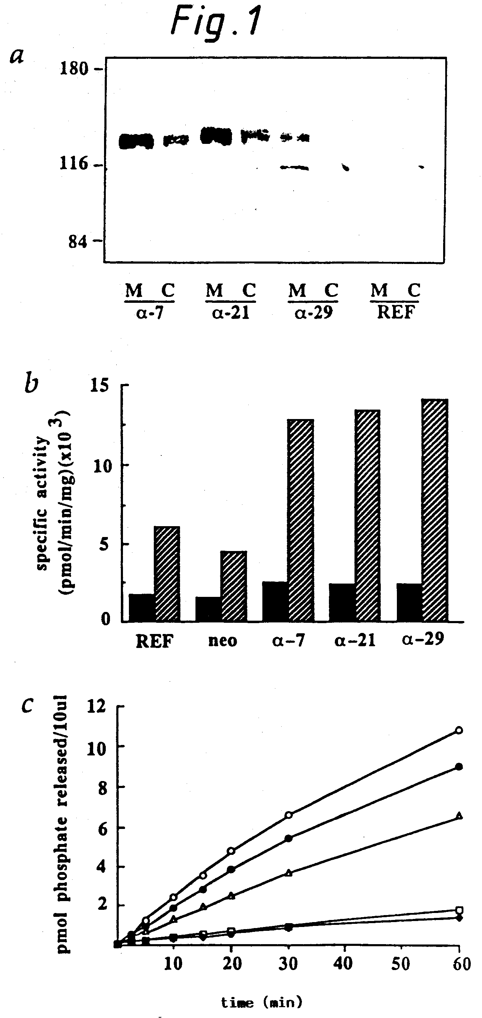

Figure 1 shows overexpression of active PTPα. a, Western blotting with anti-PTPα antiserum of solubilised membrane (M) and cytosolic (C) fractions from parental cells (REF) and cells transfected with PTPα cDNA (α-7, α-21, α-29). b, PTPase specific activities of cytosolic (solid bars) and solubilised membrane (hatched bars) fractions measured towards the phosphotyrosyl-peptide RR-src. Fractions from control transfected cells (REF-neo) are designated as neo. c, PTPase activity of immunoprecipitated PTPα from parental REF (□), control transfected REF-neo (◆) and PTPα-transfected (●, α-7; ○, α-21; △ , α-29) cells.

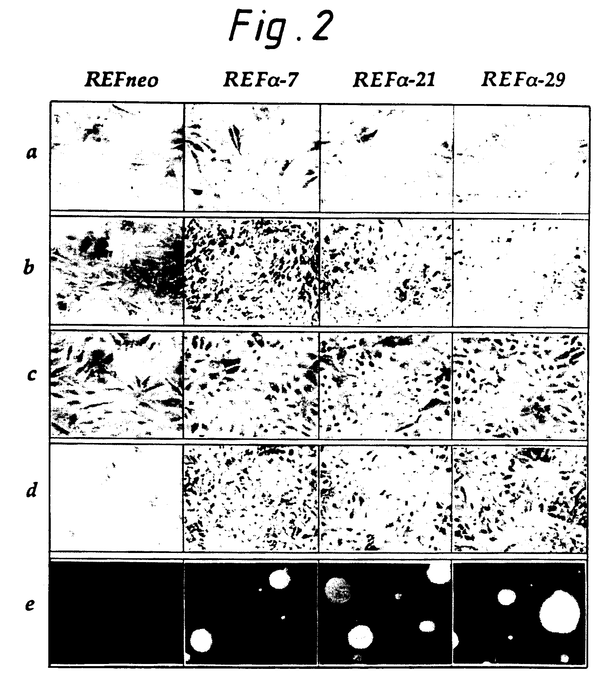

Figure 2 shows the morphologies of control REF-neo and PTPα-overexpressing cells. a, Subconfluent and b, four day post-confluent cells in DME medium with 10% fetal calf serum (FCS) (320X). c, 24 h after changing to Dulbecco's minimum essential (DME) medium with 0.1% FCS (320X). d, 72 h after changing to DME medium with 0.1% FCS (320X). e, Colonies on soft agar with serum-free DME medium (32X).

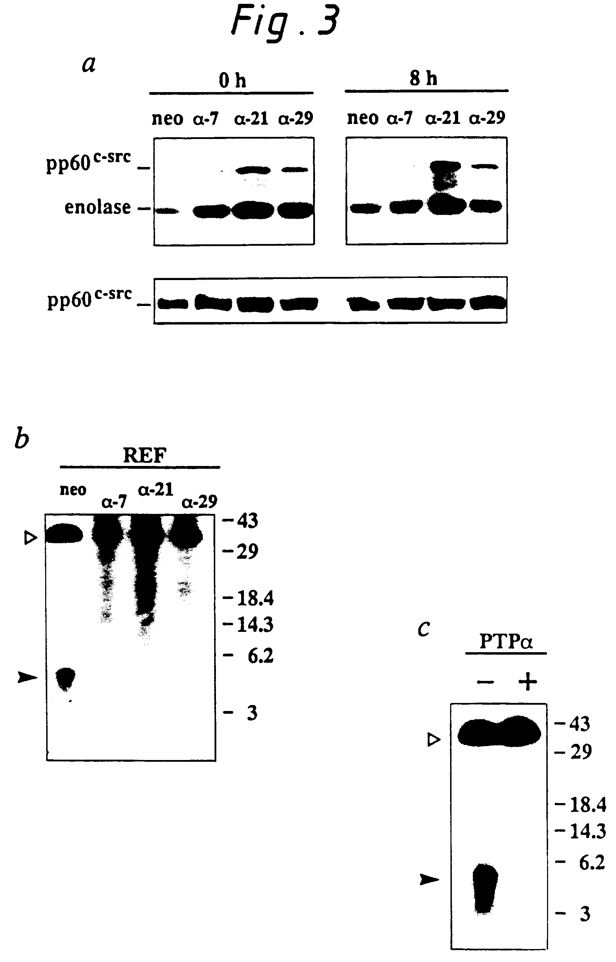

Figure 3 shows the protein kinase activity, immunoblotting and cyanogen bromide phosphopeptide analysis of pp60^{c-src}. Cells maintained in medium with 0.1% FCS for 48 h were harvested (0 h) or treated with 10% FCS for 8 h prior to harvesting (8 h). Lysates of control REF-neo cells (neo) and PTPα-overexpressing (α-7, α-21, α-29) cells were equalised for protein and immunoprecipitated with anti-src monoclonal antibody 327. a, Top; A portion of each immunoprecipitate was incubated with the exogenous substrate enolase and [γ-³²P]ATP and the mixture resolved on 10% sodium dodecyl sulphate-polyacrylamide gel electrophoresis (SDS-PAGE) followed by autoradiography. Bottom; Another portion of each immunoprecipitate was run on 10% SDS-PAGE, immunoblotted and probed with antibody 327 to measure pp60^{c-src} levels. b, Cyanogen bromide phosphopeptides of immunoprecipitated pp60^{c-src} from lysates of cells maintained in medium with 0.1% FCS for 48 h and then metabolically labelled with ³²P-orthophosphate for 16 h. c, as in b except that pp60^{c-src} immunoprecipitated from quiescent control REF-neo cells was incubated without (minus) or with (plus) the bacterially expressed and purified intracellular region of PTPα prior to digestion with cyanogen bromide. In b and c, the position of the 32 kDa phosphopeptide is designated by the open arrow and the position of the 4 kDa phosphopeptide containing Tyr 527 is designated by the closed arrow.

### Example 1

1. To facilitate cloning of the full-length PTPα cDNA¹⁶ into the BamH1-HindIII site of the expression vector pXJ41¹⁷, a 570 bp fragment of the 5' portion of the gene was first amplified by PCR. A BamH1 site (GGATCC) and an optimal Kozak consensus sequence (CCACC) were added to the 5' oligonucleotide (5'-AAGGATCCAACCACCATGGATTCCTGG-3') and the 3' oligonucleotide contained in the unique Ec1XI restriction site in the PTPα cDNA. The fidelity of amplification was confirmed by sequencing.
   The PCR fragment was cleaved with BamH1 and Ec1XI and ligated with a Ec1XI-HindIII fragment containing the rest of the PTPα coding sequence, and this was inserted into a BamH1-HindIII site in the expression vector pXJ41 to give pXJ41-PTPα. The neo gene in a pMAM-neo vector (Pharmacia) was excised with BamH1 and blunt-ended by filling with Klenow. It was inserted into the pXJ41-PTPα and pXJ41 vectors which had been cut at a Sa1I site (downstream of the PTPα insert in the former) and likewise blunt-ended. The REF cells (about 10⁶ per 15 cm dish) were transfected with 20 µg of either pXJ41-PTPα-neo or pXJ41-neo expression vector using the calcium phosphate method ¹⁸.
   After 16 h the cells were washed with DME medium (containing 4.5 g/l glucose and penicillin/streptomycin) and incubated with DME medium containing G418 (500 µg/ml) and 10% FCS. The medium was changed every four days, and colonies were isolated after 21 days. To prepare cell membrane and cytosolic fractions, confluent cells were rinsed twice with cold phosphate-buffered saline, scraped into harvesting buffer (50mM Tris-Cl pH 7.6, 150mM NaCl, 2mM PMSF, 10 µg/ml aprotinin) and pelleted by low speed centrifugation. After resuspension of the pellets in harvesting buffer and brief sonication to lyse the cells, the lysates were centrifuged at 100,000xg for 30 min at 4°C to obtain the sytosolic supernatants.
   The pellets were rinsed once with harvesting buffer, resuspended in harvesting buffer with 1% Triton X-100 and solubilised on ice for 60 min. After centrifugation at 100,000xg as above the supernatants comprised the solubilised membrane fractions. For Western blotting, 150 µg protein from each fraction was electrophoresed on 7.5% SDS-PAGE and transferred to nitrocellulose membranes in 50mM Tris base/0.56% glycine/10% methanol (80V, 75 min, 25°C). The membranes were probed with a 1:500 dilution of anti-PTPα antiserum (raised in rabbits against a peptide comprising the carboxy-terminal amino acid sequence IDAFSDYANFK of PTPα) followed by a 1:3000 dilution of goat anti-rabbit IgG conjugated to peroxidase (Sigma), and developed using the ECL system (Amersham).
   PTPase specific activity was measured at 30°C in reactions containing 50mM Mes pH 6.0, 0.5 mg/ml bovine serum albumin (BSA), 0.5mM dithiothreitol (DTT), (5µM phosphotyrosyl-RR-src peptide (RRLIEDAEY(P)AARG, corresponding to the sequence encompassing Tyr 416 of pp60^{c-src} and phosphorylated as described¹⁶) and 3.3 µg/ml solubilised membrane protein or 6.6 µg/ml cytosolic protein. Reactions were linear over the times assayed. For immunoprecipitation of PTPα, confluent cells were lysed in harvesting buffer with 1% Triton X-100 and centrifuged at 100,000xg for 30 min.
   Anti-PTPα antiserum (150 µl) was added to 6 ml of 2 mg/ml supernatant and mixed at 4°C for 16 h. Protein A-Sepharose (Pharmacia) was added and mixed at 4°C for 2 h. The immunoprecipitated pellets obtained by centrifugation were washed three times with 50mM Tris-C1 pH 7.6, 0.5M NaCl, 1mM PMSF, 10 µg/ml aprotinin and packed into small columns in the same buffer. Protein was eluted from the beads with 0.6 ml 0.1M glycine, pH 2.5 and immediately neutralised with 20 µl 1M Tris-C1, pH 8.0. The results are shown in Figures 1 and 2 and Table 1.
2. Cells were lysed in RIPA buffer¹⁹ (2 ml/15 cm dish) and the lysates centrifuged at 100,000xg for 30 min. Anti-src monoclonal antibody (Mab 327, Oncogene Science) was added to the lysates (1 µg/2ml) and mixed at 4°C for 1 h. Goat anti-mouse IgG (Sigma) was added (4 µg/2 ml) and mixed as above, followed by a one-tenth volume of Protein A cell suspension (Sigma) and mixing as above. After low speed centrifugation the immunoprecipitates were washed as described¹⁹.
   Immunoblot analysis of immunoprecipitated pp60^{c-src} levels was as described in Example 1 except immunoblots were probed with a 1:1000 dilution of 0.1 mg/ml Mab 327 followed by goat anti-mouse IgG conjugated to peroxidase (1:1000) (Sigma). The kinase activity of immunoprecipitated pp60^{c-src} was determined in 20 µl reactions containing 10mM Pipes, pH 7.0, 5mM MnC1₂, 0.5mM DTT, 2.5 µg acid-treated enolase and 10 µCi [γ-³²P]ATP at 37°C for 5 min. For cyanogen bromide phosphopeptide analysis, cells were labelled for 16 h with 1.5 mCi/ml ³²P-orthophosphate (Amersham) in phosphate-free DME containing normal DME (95:5, vol:vol) and 0.1% FCS.
   Immunoprecipitates of pp60^{c-src}, prepared from the various cells as just described, were electrophoresed on 10% SDS-PAGE and the position of ³²P-labelled pp60^{c-src} determined by autoradiography. Corresponding pieces of the gel were cut out and electroeluted in 25mM Tris, 192mM glycine, 0.1% SDS, 2% 2-mercaptoethanol at 90V for 2 h. Electroeluted protein was precipitated with 10% trichloroacetic acid with 100 µg bovine serum albumin as carrier. The precipitate was washed once with cold ethanol, dried, digested with cyanogen bromide and analysed on 20% SDS-PAGE as described²⁰. For in vitro treatment of pp60^{c-src} with PTPα prior to cyanogen bromide digestion, ³²P-labelled pp60^{c-src} immunoprecipitates were washed four times with 50mM Mes, pH 6.0, 0.5mM DTT and incubated at 30°C for 60 min in the same buffer with 0.5 µg/ml bovine serum albumin in the presence or absence of 15 µg of PTPα (intracellular domain)¹⁶. The results are shown in Figure 3.
3. Fischer rat embryo fibroblasts (REF) were transfected with the full length human receptor-like protein tyrosine phosphatase α (PTPα) cDNA in an expression vector driven by the human cytomegalovirus promoter and G418-resistant cell lines were isolated. Western blotting with anti-PTPα antibodies detected elevated levels of a protein of an expected²¹ Mᵣ of about 130 kDa in total lysates of several cell lines (not shown), and three lines (α-7, α-21, α-29) were selected for further analysis.

In accord with the transmembrane nature of PTPα solubilized membrane fractions from these cells contained much higher amounts of PTPα than those from parental cells (REF), and the overexpressed PTPα was predominantly localized to the membrane although some was detectable in the cytosolic fraction (Fig. 1a). The membrane PTPase specific activities were elevated 3-fold over those in parental and control cells transfected with expression plasmid lacking PTPα cDNA (REF-neo), while cytosolic PTPase specific activities were increased only 1.5-fold (Fig. 1b). Immunoprecipitates of PTPα from α-7, α-21 and α-29 cell lysates also had higher phosphatase activity than those prepared from control or parental cell lysates (Fig. 1c), demonstrating that the observed increases in PTPase specific activities are due to PTPα overexpression.

At subconfluency, the PTPα-overexpressing cells had a fibroblastic phenotype (Fig. 2a). At this stage the α-21 cells were beginning to exhibit a transformed phenotype with some of the cells appearing rounded and highly refractile. In contrast to the control cells, none of the PTPα-overexpressing cell lines were growth arrested through contact inhibition (Table 1). They grew in multilayers and formed foci containing refractile and disordered cells (Fig. 2b). The α-7, α-21 and α-29 cells also had a reduced requirement for serum growth factors as evidenced by their continued proliferation in medium with 0.1% FCS, whereas control cells became quiescent (Table 1).

In particular, a high proportion of α-21 cells appeared transformed within 24 h of serum deprivation (Fig. 2c). The α-7 and α-29 cells likewise appeared transformed after the concentration of FCS in the medium was reduced from 10% to 0.1%, although this phenotypic change was more apparent after 48-72 h (Fig. 2d). All three PTPα-overexpressing cells exhibited anchorage-independent growth in soft agar (with α-7 and α-21 cells showing enhanced colony formation in the absence of serum) (Fig. 2e and Table 1) and the ability to form tumours in nude mice with a short latency period (Table 1). The above features of these cells in culture in conjunction with their demonstrated in vitro tumorigenicity indicates that PTPα has transforming capability and may be oncogenic when overexpressed.

A few enzymes are activated by tyrosine dephosphorylation, such as the src family tyrosine kinases^{4, 22-24} and the cell cycle serine/threonine kinase p34^{cdc2 25-27}. Of these, certain mutant forms of pp60^{c-src}, including its oncogenic counterpart pp60^{v-src}, have transforming ability^{5-7, 28}. The activity of pp60^{c-src} in the control REF-neo and PTPα overexpressing cells was tested by immunoprecipitating pp60^{c-src} and measuring its ability to phosphorylate the exogenous substrate enolase. In cells maintained in medium with 0.1% FCS for 48 h, the kinase activity of pp60^{c-src} from α-7, α-21 and α-29 cells was increased 3- to 6-fold over that of pp60^{c-src} from control cells, and the autophosphorylation activity of pp60^{c-src} was also higher (Fig. 3a, top).

A similar activation of pp60^{c-src} was observed in cells maintained as above but treated with 10% FCS for 8 h (Fig. 3a, top) or 24 h (not shown). The amounts of pp60^{c-src} protein immunoprecipitated from all cells were comparable, as determined by probing immunoblots with an anti-src monoclonal antibody (Fig. 3a, bottom). Thus the higher activity of pp60^{c-src} in the PTPα-overexpressing cells is not due to a corresponding increase in the level of pp60^{c-src} protein.

In many instances, activation of pp60^{c-src} involves dephosphorylation of Tyr 527^{4-7, 29}. To determine whether the activation of pp60^{c-src} in PTPα-overexpressing cells could be accounted for by dephosphorylation of this residue, we immunoprecipitated pp60^{c-src} from ³²P-labelled cells and analysed its cyanogen bromide phosphopeptides. A 32 kDa phosphopeptide containing amino-terminal sites for serine and threonine phosphorylation^{20, 30} was recovered from cleaved pp60^{c-src} from both control (REF-neo) and PTPα-overexpressing cells (Fig. 3b, open arrow).

However, the pp60^{c-src} from control REF-neo cells was phosphorylated at Tyr 527 (represented by a 4 kDa phosphopeptide²⁰), while no phosphorylation of Tyr 527 was detected in pp60^{c-src} from α-7, α-21 and α-29 cells (Fig. 3b, closed arrow). A direct interaction between PTPα and pp60^{c-src} could be demonstrated in vitro, where bacterially expressed and purified PTPα was able to dephosphorylate Tyr 527 of pp60^{c-src} immunoprecipitated from quiescent control cells (Fig. 3c).

The activation state of pp60^{c-src} reflects the balance of opposing activities of a Tyr 527 kinase and phosphatase, where phosphorylation of Tyr 527 negatively regulates pp60^{c-src} activity. A specific Tyr 527 kinase has recently been identified and cloned^{31, 32}, although as yet nothing is known of the regulation of its activity. Cell cycle-regulated inhibition of such a kinase or activation of an appropriate tyrosine phosphatase, or both, could result in Tyr 527 dephosphorylation and activation of mitotic pp60^{c-src}. The unregulated occurrence of these events may lead to uncontrolled cell proliferation. In accord with this, we have shown that the constitutive overexpression of active PTPα subverts the normal control of cell growth and have identified pp60^{c-src} as a target for PTPα action.

The in vivo dephosphorylation of Tyr 527 of pp60^{c-src} in PTPα-overexpressing cells and the cellular association of both proteins with membranes³³, when coupled with the ability of purified PTPα to dephosphorylate the same site of pp60^{c-src} in vitro, suggests that pp60^{c-src} may be a direct substrate of PTPα. Since PTPα is widely expressed and is particularly abundant in brain^{21, 34} while pp60^{c-src} is expressed in most cells and at high levels in neural tissue³⁵, PTPα may function as an in vivo regulator of src kinase activity in normal cells.

**TABLE 1**

| Growth characteristics of parental, control and PTPα-overexpressing cells. | | | | | |
|---|---|---|---|---|---|
| cells | Saturation density (cells/cm²) | cell proliferation at 0.1% FCS | colonies in soft agar^{c} | | tumours in nude mice (latency)^{d} |
| | | | 10% FCS | 0% FCS | |
| REF | ND^{a} | No | 0 | 0 | 0/5 |
| REF-neo | 1.2x10⁵ | No | 0 | 0 | 0/4 |
| REFα-7 | >1.5x10⁶^{b} | Yes | 217 ± 20 | 416 ± 11 | 4/4 (5-6d) |
| REFα-21 | >1.5x10⁶ | Yes | 420 ± 18 | 789 ± 42 | 4/4 (5-6d) |
| REFα-29 | >1.5x10⁴ | Yes | 781 ± 1 | 456 ± 28 | 4/4 (5-6d) |

| | | | | | |
|---|---|---|---|---|---|
| a ND, not determined | | | | | |
| b No apparent saturation density was observed with α-7, α-21 or α-29 cells. Proliferation continued to the point where fresh medium was required every 3-5 hours and eventually these cells lifted off the dishes in sheets. | | | | | |
| c 400 cells were added to each 6 cm petri dish and colonies were counted after three weeks. Counts are the average of triplicate experiments ± standard error. | | | | | |
| d Each mouse was injected subcutaneously at one site on the back with 2x10⁵ cells. | | | | | |

### EXAMPLE 2

### Effect of sodium orthovanadate on proliferation of PTPα-overexpressing and control (REF-neo) cells

1. Cells were grown on 5 cm petri dishes in DME medium with 10% FCS for 48 h. After 48 h of growth, each dish of α-7, α-21, and α-29 cells contained about 0.4 x 10⁵ to 0.6 x 10⁵ cells, and each dish of REF-neo cells contained 1.8 x 10⁵ cells. The medium was discarded, and fresh medium containing 10% FCS and the indicated concentration (below) of sodium orthovanadate was added to the cells. After 48 h of culture in this medium the cells from two replicate dishes were harvested and counted. The results are shown in Table 2 below. They demonstrate that vanadate inhibits the proliferation of the PTPα-overexpressing cells (α-7, α-21, α-29) in a dose-dependent manner while having relatively little effect on that of the control cells (REF-neo).

**TABLE 2**

| Vanadate conc (µM) | REF-neo | Number of cells/5 cm dish | | |
|---|---|---|---|---|
| | | α-7 | α-21 | α-29 |
| 0 | 1.20 x 10⁶ | 1.80 x 10⁶ | 1.60 x 10⁶ | 1.72 x 10⁶ |
| 5 | 1.10 x 10⁶ | 1.72 x 10⁶ | 1.42 x 10⁶ | 1.62 x 10⁶ |
| 30 | 1.04 x 10⁶ | 1.41 x 10⁶ | 7.00 x 10⁵ | 7.20 x 10⁵ |
| 60 | 9.90 x 10⁵ | 1.18 x 10⁶ | 3.70 x 10⁵ | 4.70 x 10⁵ |
| 120 | 7.60 x 10⁵ | 5.80 x 10⁵ | 1.60 x 10⁵ | 2.10 x 10⁵ |

2. Cells (control cells = REF-neo, PTPα-overexpressing cells = α-21) were grown on 5 cm petri dishes in DME medium with 10% FCS for 48 h. The medium was discarded, and fresh medium containing 10% FCS and the indicated concentration (below) of sodium orthovanadate was added to the cells. After 72 h of culture in this medium the cells from two replicate dishes were harvested and counted. The results are shown in Table 3 below and are essentially the same as in 1 above.

**TABLE 3**

| Vanadate conc (µM) | Number of cells/5cm dish | |
|---|---|---|
| | REF-neo | α-21 |
| 0 | 1.84 x 10⁶ | 1.38 x 10⁶ |
| 5 | 1.90 x 10⁶ | 1.20 x 10⁶ |
| 30 | 1.80 x 10⁶ | 4.80 x 10⁵ |
| 60 | 1.60 x 10⁶ | 2.60 x 10⁵ |
| 120 | 6.70 x 10⁵ | 1.76 x 10⁵ |

3. PTPα-overexpressing cells (α-21) were grown for 48 h (to about 60% confluency) in DME medium containing 10% FCS. The medium was removed and replaced with medium containing the indicated concentration of sodium orthovanadate (below) and either 10% (sets #1 and #3) or 0.1% FCS (set #2). The cells were cultured for 48 h and counted (sets #1 and 2), or the medium was removed from set #3 and replaced with medium containing 10% FCS without sodium orthovanadate and cultured for another 48 h before counting. The results are shown in Table 4 below and demonstrate that the proliferation of α-21 cells grown in either 10% or 0.1% FCS is similarly affected by vanadate in a dose-dependent manner and that vanadate is not irreversibly toxic to these cells since they will resume proliferation once vandate is removed from the medium as may be appreciated by comparison of the cell numbers in sets #1 and 3.

**TABLE 4**

| Vanadate conc (µM) | set #1 (+10% FCS) | set #2 (+0.1% FCS) | set #3 |
|---|---|---|---|
| 0 | 1.62 x 10⁶ | 1.52 x 10⁶ | 5.12 x 10⁶ |
| 5 | 1.30 x 10⁶ | 1.18 x 10⁶ | 2.90 x 10⁶ |
| 30 | 5.00 x 10⁵ | 5.40 x 10⁵ | 1.46 x 10⁶ |
| 60 | 3.20 x 10⁵ | 3.80 x 10⁵ | 9.60 x 10⁵ |
| 120 | 1.80 x 10⁵ | 2.00 x 10⁵ | 5.20 x 10⁵ |

### Example 3: Effect of sodium orthovanadate on the colony forming ability of PTPα-overexpressing α-21 cells.

The α-21 cells were grown in soft agar in the presence of the indicated concentration of sodium orthovanadate (below) on duplicate dishes. After 9 days the colonies were counted on each dish and the approximate size of the colonies noted. The results are shown in Table 5 below and demonstrate the reduced ability of the PTPα-overexpressing cells to form colonies in soft agar in the presence of vanadate.

**TABLE 5**

| Vanadate conc (µM) | Number of colonies | | | |
|---|---|---|---|---|
| | Exp 1 | Exp 2 | Average | Colony size |
| 0 | 1650 | 1461 | 1556 | +++ |
| 5 | 1127 | 1392 | 1260 | +++ |
| 10 | 1458 | 934 | 1196 | +++ |
| 20 | 692 | 641 | 667 | ++ |
| 40 | 312 | 271 | 292 | + |
| 80 | 90 | 73 | 82 | +/- |

### Example 4: Determination whether vanadate can block or inhibit tumourigenesis caused by REF α-21 PTPα-overexpressing cells.

The α-21 cells were grown in DME medium containing 10% FCS and then in DME medium with 0.1% FCS in the presence of 50 µM sodium orthovanadate for 48 h. The cells were then harvested by trypsinisation, centrifuged to pellet the cells, and the cell pellet resuspended in DME containing 50 µM sodium orthovanadate. Other α-21 cells were grown and prepared in a similar manner, except in the absence of sodium orthovanadate throughout. The resuspended cells (plus or minus vanadate) were subcutaneously injected into nude mice (2 x 10⁶ cells/mouse, 4 mice for each treatment), and nine days later the tumour size was measured. The results below show that tumours arising from cells grown and injected in the presence of vanadate are on average 2.6 times smaller than those arising from cells grown and injected in the absence of vanadate.

| cells + vanadate | tumour size | average tumour size |
|---|---|---|
| mouse 1: | 0.30 x 0.8 cm = 0.24 cm² | 0.618 cm² |
| mouse 2: | 0.70 x 1.5 cm = 1.05 cm² | |
| mouse 3: | 0.40 x 0.7 cm = 0.28 cm² | |
| mouse 4 | 0.75 x 1.2 cm = 0.90 cm² | |

| cells - vanadate | tumour size | average tumour size |
|---|---|---|
| mouse 5: | 0.80 x 1.4 cm = 1.12 cm² | 1.603 cm² |
| mouse 6: | 0.75 x 2.2 cm = 1.65 cm² | |
| mouse 7: | 0.90 x 1.8 cm = 1.62 cm² | |
| mouse 8: | 0.88 x 2.3 cm = 2.02 cm² | |

### REFERENCES

1. Chackalaparampil, I. & Shalloway, D. Cell 52, 801-810 (1988).
2. Bagrodia, S., Chackalaparampil, I., Kmiecik, T.E. & Shalloway, D. Nature 349, 172-175 (1991).
3. Kaech, S. Covik, L., Wyss, A. & Ballmer-Hofer, K. Nature 350, 431-433 (1991).
4. Cooper, J.A., Gould, K.L., Cartwright, C.A. & Hunter, T. Science 231, 1431-1434 (1986).
5. Kmiecik, T.E. & Shalloway, D. Cell 49, 65-73 (1987).
6. Piwnica-Worms, H., Saunders, K.B. Roberts, T.M., Smith, A.E. & Cheng S.H. Cell 49, 75-82 (1987).
7. Cartwright, C.A., Eckhart, W., Simon, S. & Kaplan, P.L. Cell 49, 83-91 (1987).
8. Fischer, E.H., Charbonneau, H . & Tonks, N.K. Science 253, 401-406 (1991).
9. La Forgia, S., Morse, B., Levy, J., Barnea, G., Cannizaro, L.A., Li, F., Nowell, P.C., Boghosian-Sell, L., Glick, J., Weston, A., Harris, C.C., Drabkin, H., Patterson, D., Croce, C.M., Schlessinger, J. & Huebner, K. Proc. Natl. Acad. Sci. USA 88, 5036-5040 (1991).
10. Rosen, N., Bolen, J.B. Schwartz, A.M. Cohen, P., DeSeau, V. & Israel, M.A. J.Biol. Chem. 261, 13754-13756 (1986).
11. Bolen, J.B., Veillette, A., Schwartz, A.M., DeSeau, V. and Rosen, N. Proc. Natl. Acad. Sci. USA 84, 2251-2255 (1987).
12. Cartwright, C.A., Kamps M.P., Meister, A.I., Pipos, J.M. and Eckhart, W. J. Clin. Invest. 83,2025-2033 (1989).
13. Cartwright, C.A., Meister, A.I. and Eckhart, W. Proc. Natl. Acad. Sci. USA 87, 558-562, 1990.
14. Fanning, P., Bulovas, K., Saini, K.S. Libertino, J.A. Joyce, A.D. and Summerhayes, I.C. Cancer Research, 52, 1457-1462, 1992.
15. Miller, A.D. Nature 357, 455-460, 11 June 1992 and references cited therein.
16. Wang, Y. & Pallen, C.J. EMBO J. 10, 3231-3237 (1991).
17. Xiao, J.H., Davidson, I., Matthes, H., Garnier, J.M. & Chambon, P. Cell 65, 551-568 (1991).
18. Graham, F.L. & Van Der Eb, J. Virology 52, 456-467 (1973).
19. Gould, K.L. & Hunter, T. Mol. Cell. Biol. 8, 3345-3356 (1988).
20. Bolen, J.B., Veillette, A., Schwartz, A.M. DeSeau, V. & Rosen, N. Oncogene Res. 1, 149-168 (1987).
21. Sap, J., D'Eustachio, P., Givol, D. & Schlessinger, J. Proc. Natl. Acad. Sci. USA 87, 6112-6116 (1990).
22. Marth, J.D., Cooper, J.A., King, C.S., Ziegler, S.F., Tinker, D.A., Overell, R.W., Krebs, E.G. & Perlmutter, R.M. Mol. Cell. Biol. 8, 540-540 (1988).
23. Amrein, K.E. & Sefton, B.M. Proc. Natl. Acad. Sci. USA 85, 4247-4251 (1988).
24. Kawakami,T., Kawakami, Y., Aaronson, S.A. & Robbins, K.C. Proc. Natl. Acad. Sci. USA 85, 3870-3874 (1988).
25. Dunphy, W.G. & Newport, J.W. Cell 58, 181-191 (1989).
26. Morla, A.O., Draetta, G., Beach, D. & Wang, J Y.J. Cell 58, 193-204 (1990).
27. Gould, K.L. & Nurse, P. Nature 342, 39-45 (1989).
28. Parker, R.C., Varmus, H.E. & Bishop, J.M. Cell 37, 131-139 (1984).
29. Courtneige, S.A. EMBO J. 4, 1471-1477 (1985).
30. Shenoy, S., Choi, J.-K., Bagrodia, S., Copeland, T.D., Maller, J.L. & Shalloway, D. Cell 57, 763-774 (1989).
31. Nada, S., Okada, M., MacAuley, A., Cooper J.A. & Nakagawa, H. Nature 351, 69-72 (1991).
32. Okada, M. & Nakagawa, H. J. Biol. Chem. 264, 20886-20893 (1989).
33. Courtneige, S.A., Levinson, A.D. & Bishop, J.M. Proc. Natl. Acad. Sci. USA 77, 3783-3787 (1980).
34. Matthews, R.J., Cahir, E.D. & Thomas, M.L. Proc. Natl. Acad. Sci. USA 87, 4444-4448 (1990).
35. Cotton, P.C. & Brugge, J.S. Mol. Cell. Biol. 3, 1157-1162 (1983).

## Claims

1. An inhibitor of protein tyrosine phosphatase α for use in the treatment of a tumour exhibiting an elevated level of pp60^{c-src} kinase activity.

2. An inhibitor according to claim 1, in which the tumour is human colon carcinoma.

3. An inhibitor according to claim 1 or 2, which is a chemical compound.

4. An inhibitor according to claim 1 or 2, which is an antibody.

5. An inhibitor according to claim 1 to 2, which is a cDNA encoding a mutant protein tyrosine phosphatase α.

6. Use of an inhibitor of protein tyrosine phosphatase α in the manufacture of a medicament for use in the treatment of a tumour exhibiting an elevated level of pp60^{c-src} kinase activity.

## Patentansprüche

1. Inhibitor der Proteintyrosinphosphatase alpha zur Verwendung bei der Behandlung eines Tumors, der einen erhöhten Grad der pp60^{c-src}-Kinaseaktivität zeigt.

2. Inhibitor nach Anspruch 1, worin der Tumor ein humanes Dickdarmkarzinom ist.

3. Inhibitor nach Anspruch 1 oder 2, welcher eine chemische Verbindung ist.

4. Inhibitor nach Anspruch 1 oder 2, welcher ein Antikörper ist.

5. Inhibitor nach Anspruch 1 oder 2, welcher eine cDNA ist, die für eine mutante Proteintyrosinphosphatase alpha codiert.

6. Verwendung eines Inhibitors der Proteintyrosinphosphatase alpha bei der Herstellung eines Medikaments zur Verwendung bei der Behandlung eines Tumors, der einen erhöhten Grad der pp60^{c-src}-Kinaseaktivität zeigt.

## Revendications

1. Inhibiteur de tyrosine protéine-phosphatase α pour utilisation dans le traitement d'une tumeur présentant un niveau élevé d'activité kinase pp60^{c-src}.

2. Inhibiteur selon la revendication 1, où la tumeur est un carcinome du colon humain.

3. Inhibiteur selon la revendication 1 ou 2, qui est un composé chimique.

4. Inhibiteur selon la revendication 1 ou 2, qui est un anticorps.

5. Inhibiteur selon la revendication 1 ou 2, qui est un ADNc codant pour une tyrosine protéine-phosphatase α mutante.

6. Utilisation d'un inhibiteur de tyrosine protéine-phosphatase α dans la préparation d'un médicament pour utilisation dans le traitement d'une tumeur présentant un niveau élevé d'activité kinase pp60^{c-src}.
